(19) Europäisches Patentamt
European Patent Office
Office européen des brevets

(11) Numéro de publication: 0 135 433
B1

(12) **FASCICULE DE BREVET EUROPEEN**

(45) Date de publication du fascicule du brevet: 18.01.89

(21) Numéro de dépôt: 84401676.6

(22) Date de dépôt: 16.08.84

(51) Int. Cl.[4]: A 61 K 31/235, C 07 C 69/76, C 07 C 65/21, C 07 C 65/01, C 07 C 69/92 // C07C33/20, C07C121/52, C07C79/22, C07C79/35, C07C87/50

(54) **Dérivés de l'acide benzoique pour utilisation comme médicament et utilisation de ces dérivés comme agents conservateurs ou désinfectants.**

(30) Priorité: 18.08.83 FR 8313445

(43) Date de publication de la demande: 27.03.85 Bulletin 85/13

(45) Mention de la délivrance du brevet: 18.01.89 Bulletin 89/03

(84) Etats contractants désignés: BE CH DE FR GB IT LI LU NL SE

(56) Documents cités:
FR-A-2 241 296
FR-A-2 272 647
GB-A-1 050 136

(73) Titulaire: SANOFI
40, Avenue George V
F-75008 Paris (FR)

(72) Inventeur: Demarne, Henri
Le Florence 65 avenue Major Flandres
F-34000 Montpellier (FR)
Inventeur: Filhol, Robert
Château d'Alco Bâtiment 7 rue des Avants-Monts
F-34000 Montpellier (FR)
Inventeur: Mossé, Madeleine
La Chanterelle Chemin du Réservoir du Montmaur
F-34100 Montpellier (FR)

(74) Mandataire: Gillard, Marie-Louise et al
Cabinet Beau de Loménie 55, Rue d'Amsterdam
F-75008 Paris (FR)

EP 0 135 433 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention a pour objet des dérivés de l'acide benzoïque pour utilisation comme médicament. Elle concerne également l'utilisation de ces dérivés comme agents conservateurs ou désinfectants.

Lesdits dérivés de l'acide benzoïque, selon l'invention, ont pour formule générale:

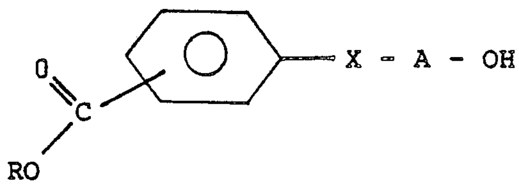

(I)

dans laquelle:

— A représente une chaîne alkylene droite ou ramifiée comprenant de 3 à 4 atomes de carbone;
— X représente l'atome d'oxygene ou une liaison directe;
— R représente l'hydrogène ou un groupement alkyle de 2 à 6 atomes de carbone éventuellement substitué par une fonction alcool.

La présente invention concerne également l'utilisation, dans des compositions à usage antiseptique, des sels convenables de (I).

Ces composés présentent une activité antimicrobienne, ils peuvent notamment être utilisés comme médicaments antiseptiques à usage humain ou vétérinaire ou comme désinfectants sur des surfaces inertes. Ils peuvent également être utilisés comme conservateurs.

Les composés (I), dans lesquels X représente une liaison directe, peuvent être préparés selon la méthode décrite dans J. Med. Chem., 1983, vol. 26, pages 335—341, à partir d'un acide toluique. Certains sont également déjà connus par GB—A—1050136.

On peut également les préparer à partir des nitrophénylalcanols ou des nitrophénoxyalcanols (IV). Par hydrogénation catalytique de (IV), on obtient les dérivés de l'aniline correspondants (V), puis par addition du nitrite de sodium en milieu acide, les diazoniums. L'action du cyanure cuivreux conduit ensuite, par la réaction de Sandmeyer, aux dérivés du benzonitrile (VI). Enfin, les composés (I) sont synthétisés par des réactions connues en elles-mêmes et éventuellement transformés en un sel convenable.

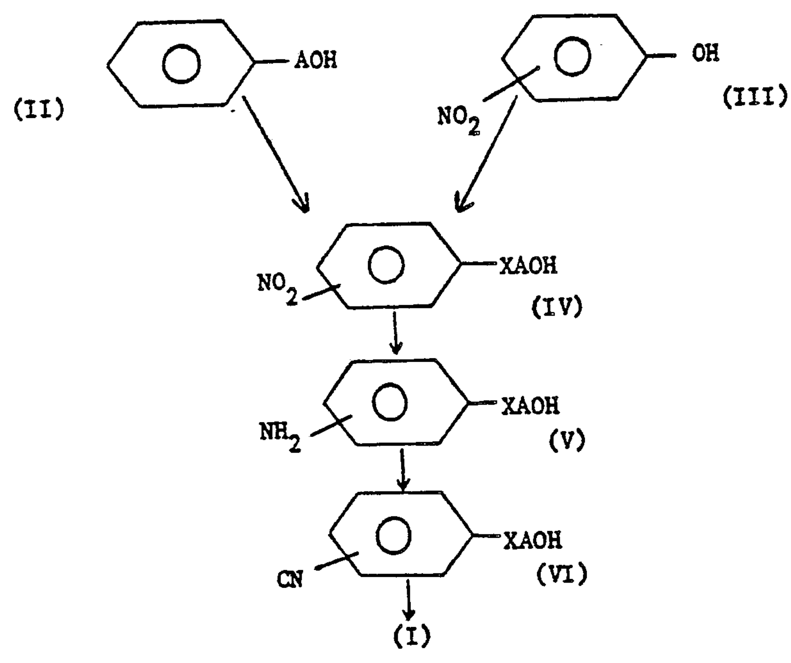

Lorsque X représente une liaison directe, on prépare le nitrophénylalcanol (IV) à partir du phénylalcanol (II). Au préalable, on protège le groupement hydroxyle par acétylation au moyen du chlorure d'acétyle. Après la nitration par l'acide nitrique fumant, l'alcool est libéré par action du méthanol chlorhydrique.

Les phénylalcanols (II) sont accessibles commercialement lorsqu'il s'agit d'alcools linéaires. Sinon, ils peuvent être préparés par diverses méthodes. Par exemple, les phénylalcanols secondaires sont préparés à

partir d'un phénylacétaldéhyde par action d'un dérivé magnésien suivi d'une hydrolyse:

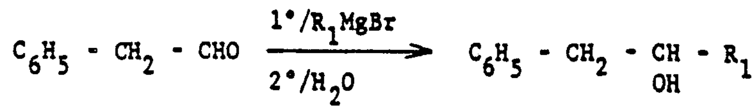


(II)'

$R_1$: alkyle de 1 à 8 carbones

Les phénylalcanols ramifiés primaires sont obtenus à partir du cyanure de benzyle:

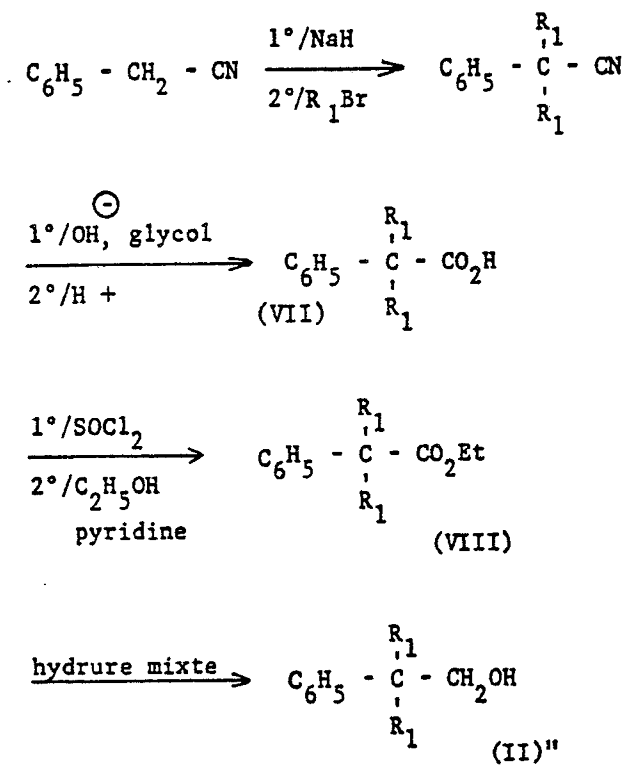


(VII) (VIII) (II)"

Après avoir fait réagir l'hydrure de sodium en milieu anhydre, on peut additionner un halogénure d'alkyle, par exemple le bromure, pour obtenir un phényl-acétonitrile dialkylé symétrique. La transformation de ce composé en acide s'effectue par action d'une base en milieu alcoolique, suivie d'une acidification.

L'action du chlorure de thionyle puis de l'alcool éthylique en milieu anhydre en présence d'un catalyseur, par exemple la pyridine ou la diméthylaminopyridine, permet d'obtenir l'ester éthylique (VII). On prépare ensuite l'alcool correspondant (II)" par réduction à l'aide d'un hydrure mixte dans un solvant anhydre.

Lorsque X représente l'oxygène, on prépare le nitrophénoxyalcanol (IV) à partir du nitrophénol (III). L'action sur (III) d'un dihalogénure d'alkyle en milieu basique permet d'obtenir un halogénure de nitrophénoxyalkyle (IX). Ce produit est acétylé en milieu acide puis (IV) est libéré par saponification.

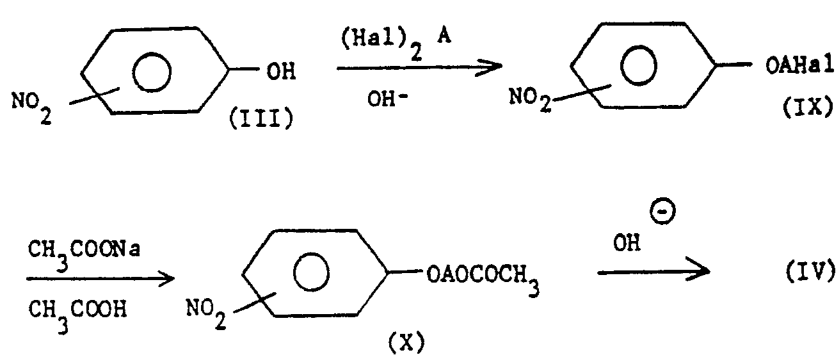


(III) (IX) (X) (IV)

Dans tous les cas, les composés (I) où R = alkyle ou hydroxyalkyle sont obtenus à partir des composés (I) où R = H par les procédés habituels d'estérification et en particulier par action de l'alcool R—OH sur l'acide en présence d'acide fort comme catalyseur ou par action d'un halogénure R Hal sur le sel de sodium de l'acide.

Les exemples suivants illustrent l'invention sans toutefois la limiter. Lorsque le produit obtenu est sous forme d'huile, il est caractérisé par son spectre de résonance magnétique nucléaire (RMN). Celui-ci est enregistré à 60 MHz dans le deutérochloroforme, en prenant comme étalon interne l'hexaméthyldisiloxane.

Pour décrire le spectre, les abréviations suivantes sont utilisées:

S : singulet
D : doublet
T : triplet
Q : quadruplet
M : multiplet
J : constante de couplage.

Exemple 1

*Acide (hydroxy-3 propyl)-4 benzoïque : SR 41323.*

a) — (Nitro-4 phényl)-3 propanol-1.

A 171,5 g de phényl-3 propanol-1, on ajoute en une 1 h sous agitation, 95 ml de chlorure d'acétyle. On chauffe 2 h à reflux, l'acide chlorhydrique qui se dégage et l'excès de chlorure d'acétyle sont éliminés. Lorsque le milieu réactionnel est revenu à la température ambiante, on le verse goutte à goutte, en agitant, sur 800 ml d'acide nitrique fumant (d = 1,49) refroidi à −25°C, l'addition dure 1 h pendant laquelle la température est maintenue entre −15°C et −20°C. On verse ensuite sur 1,5 l d'eau additionnée de glace pilée puis on extrait 3 fois à l'éther, lave 3 fois à l'eau, 3 fois avec une solution de carbonate de sodium à 10%, puis 3 fois à l'eau. Les phases éthérées sont séchées sur sulfate de magnésium puis évaporées à sec sous pression réduite. Le résidu est repris dans 800 ml de méthanol puis on fait barboter de l'acide chlorydrique gazeux 1 h à 0°C, on chauffe ensuite pendant 14 h à reflux. Après évaporation du solvant, on reprend dans le mélange eau-éther, décante la phase aqueuse, lave 2 fois à l'eau, 3 fois avec une solution saturée de bicarbonate de sodium puis 3 fois à l'eau; la phase éthérée est ensuite séchée sur sulfate de magnésium, puis évaporée à sec sous pression réduite.

On obtient 259 g d'une huile orangée que l'on purifie par chromatographie sur 3 kg de gel de silice dans le chloroforme; on récupère 218 g d'huile orangèe; Rendement 95%.

b) — (Hydroxy-3 propyl)-4 aniline.

218 g de (nitro-4 phényl)-3 propanol-1 sont dissous dans 500 ml de méthanol, on ajoute 10 g de palladium à 10% sur charbon préalablement humectés avec 10 ml d'eau. L'hydrogénation s'effectue sous une pression de 40 bars et sous agitation, elle dure 1 h 30. On filtre ensuite sur céilite, rince au méthanol, évapore à sec sous pression réduite pour obtenir 168 g d'une huile marron. Celle-ci est purifiée par 3 chromatographies successives sur 6 kg d'alumine au total, en tuilisant le dichlorométhane comme éluant. On obtient 49,2 g d'une poudre marron clair; Fc = 43—45°C; Rendement: 27%.

c) — (Hydroxy-3 propyl)-4 benzonitrile.

49,07 g du produit obtenu précédemment sont versés dans 87 ml d'acide chlorhydrique concentré additionné de 400 g de glace pilée. En maintenant la température entre 0°C et 5°C, on ajoute goutte à goutte une solution de 23,15 g de nitrile de sodium dans 80 ml d'eau puis, après 10 min d'agitation, on neutralise avec 300 ml de solution à 10% de carbonate de sodium.

On prépare par ailleurs une solution de cyanure cuivreux: 40,35 g de chlorure cuivreux sont mis en suspension dans 150 ml d'eau, on ajoute une solution de 54 g de cyanure de sodium dans 80 ml d'eau. On observe un dégagement de chaleur, le chlorure cuivreux se dissout, la solution est décolorée. A cette solution refroidie à 0°C et addittionnée de 200 ml de benzène, on ajoute goutte à goutte pendant 40 min en agitant voilemment, la solution de diazonium refroidie à 0°C. Après 40 min d'agitation supplémentaire, on laisse revenir à température ambiante, sous agitation, puis on chauffe à 50°C sans agiter et on ramène à température ambiante.

On extrait 3 fois à l'éther, lave 2 fois à l'eau, puis avec une solution saturée de chlorure de sodium. Les phases éthérées sont séchées sur sulfate de magnésium et évaporées à sec sous pression réduite. On obtient 51 g d'une huile marron foncé. Celle-ci est purifiée par chromatographie sur 1 500 g de gel de silice, la colonne est préparée dans du toluène et l'éluant est un mélange toluène-éther (9/1 en volume). On obtient 41,6 g produit pur sous forme d'huile rouge; Rendement: 79%.

Le produit est caractérisé par son spectre de RMN:

2H entre 1,7 et 2,2 ppm (M, $—CH_2—CH_2—CH_2—OH$)
1H à 2,4 ppm (S, —OH)
2H à 2,8 ppm (T, J = 7 Hz, $CN—C_6H_4—CH_2—CH_2—$)
2H à 3,6 ppm (T, J = 6 Hz, $—CH_2—CH_2—OH$)
2H à 7,3 ppm (D, J = 9 Hz, H ortho $CH_2$)
2H à 7,6 ppm (D, J = 9 Hz, H ortho CN)

d) — SR 41323

41 g du produit précédent sont dissous dans 150 ml d'alcool à 95°, on additionne 15 g de soude puis on chauffe à reflux 17 h sous agitation, après refroidissement, on évapore à sec sous pression réduite, le résidu est repris dans l'eau et acidifié à pH voisin de 1 par de l'acide chlorhydrique concentré. On extrait 2 fois à l'éther, lave 2 fois à l'eau puis avec une solution saturée de chlorure de sodium. Les phases éthérées sont ensuite séchées sur sulfate de magnésium et évaporées à sec sous pression réduite. Les cristaux jaunes obtenus sont recristallisés dans un mélange éther-hexane.

On obtient 33,7 g de poudre crème; Fc = 138—141°C.

Exemple 2

*(Hydroxy-3 propyl)-4 benzoate d'éthyle : SR 41324*

13,5 g du produit précédemment obtenu (SR 41323) sont dissous dans 200 ml d'éthanol absolu, on ajoute goutte à goutte, en agitant, 6 ml de chlorure de thionyle. Lorsque le milieu réactionnel est revenu à la température ambiante, on chauffe à reflux 6 h sous agitation puis on abandonne une nuit à température ambiante. Les solvants sont évaporés sous pression réduite, puis on extrait 3 fois à l'éther, lave 2 fois avec une solution saturée de bicarbonate de sodium, 2 fois à l'eau puis avec une solution saturée de chlorure de sodium. Les phases éthérées sont ensuite séchées sur sulfate de magnésium et évaporées à sec sous pression réduite.

On obtient 15,43 g correspondant au produit attendu caractérisé par son spectre de RMN.

3H à 1,3 ppm (T, J = 7 Hz, $—CO_2—CH_2—CH_3$)
3H entre 1,5 et 2,2 ppm (massif, $HO—CH_2—CH_2—CH_2$)
2H à 2,7 ppm (T, J = 7 Hz, $HO—CH_2—CH_2—CH_2—C_6H_4$)
2H à 3,6 ppm (T, J = 6 Hz, $HO—CH_2CH_2—CH_2—$)
2H à 4,3 ppm (Q, J = 7 Hz, $—CO_2—CH_2—CH_3$)
2H à 7,2 ppm (D, J = 9 Hz, H ortho $CH_2$)
2H à 7,9 ppm (D, J = 9 Hz, H ortho $CO_2$)

Exemple 3

*Acide (hydroxy-2 butyl)-4 benzoïque: CM 41074.*

a) — Phényl-1 butanol-2.

A 2,92 g de magnésium en tournures, on ajoute sous azote, goutte à goutte, à une vitesse suffisante pour maintenir un léger reflux, une solution de 7,5 ml de bromure d'éthyle dans 50 ml d'éther anhydre. Toujours sous azote, on agite pendant 2 h à température ambiante, puis on ajoute goutte à goutte 9,4 ml de phénylacétaldéhyde et on laisse 2 h à température ambiante sous agitation.

On décompose ensuite sur 200 ml de chlorure d'ammonium à 20% refroidi à 0°C et on extrait 3 fois à l'éther. Après 3 lavagès à l'eau, les phases éthérées sont séchées sur sulfate de magnésium et évaporées à sec sous pression réduite. On obtient 12,2 g d'une huile légèrement jaune.

b) — CM 41074.

En utilisant ensuite les procédés décrits à l'exemple 1, on prépare le CM 41074 qui est recristallisé dans un mélange étherhexane; Fc = 103—106°C.

Exemple 4

*(Hydroxy-2 butyl)-4 benzoate d'éthyle: CM 41075.*

Ce produit est préparé à partir de l'acide CM 41074, il est caractérisé par son spectre de RMN.

3H à 0,9 ppm (T, J = 7 Hz, $CH_3—CH_2—CH(OH)—CH_2—$)
6H entre 1,1 et 1,7 ppm (massif, $CH_3—CH_2—CH(OH)$, $—CO_2—CH_2—CH_3$)
2H entre 2,6 et 2,9 ppm (M, $CH(OH)—CH_2—C_6H_4—$)
1H entre 3,5 et 3,9 ppm (M, $—CH_2—CH(OH)—CH_2$)
2H à 4,3 ppm (Q, J = 7 Hz, $—CO_2—CH_2—CH_3$)
2H à 7,3 ppm (D, J = 9 Hz, H ortho $CH_2$)
2H à 8 ppm (D, J = 9 Hz, H ortho $CO_2$)

Exemple 5

*Acide (hydroxy-4 butyloxy)-4 benzoïque: CM 40841*

a) — (Nitro-4 phénoxy)-1, bromo-4 butane.

A une solution de nitro-4 phénol dans 275 ml d'eau, on ajoute 83 ml de dibromo-1,4 butane puis, goutte à goutte, 49,5 ml de soude 10 N sous agitation. On chauffe à reflex sous agitation pendant 24 h.

Après refroidissement, on extrait 3 fois à l'éther, lave 6 fois à la soude normale, puis 3 fois à l'eau. Les phases éthérées sont séchées sur sulfate de sodium et évaporées, on filtre l'insoluble. Le filtrat est évaporé à sec et le résidu est tiré sous vide (6.67 Pa (0,05 mm Hg)). Après trituration dans l'hexane, on obtient des cristaux que l'on filtre, lave à l'hexane et sèche sous vide au dessiccateur.

On obtient 75 g d'un produit pâteux de couleur crème; Rendement 55%.

b) — (Nitro-4 phénoxy)-1 acétyloxy-4 butane.

75 g du produit précédent sont dissous dans 80 ml d'acide acétique glacial, on ajoute 45 g d'acétate de sodium anhydre, puis on chauffe à reflux pendant 15 h sous agitation. Le mélange réactionnel est versé sur 1 l d'eau glacée additionnée de 500 ml d'éther et on neutralise à pH 7,5 par du carbonate de sodium solide.

Après 3 extractions à l'éther et 3 lavages à l'eau, les phases éthérées sont séchées sur sulfate de magnésium et évaporées à sec, le résidu est tiré sous vide.

On obtient m = 70 g d'une huile orangée; Rendement: 100%.

c) — (Nitro-4 phénoxy)-1 butanol-4.

70 g du produit obtenu précédemment sont mis en solution dans 300 ml de méthanol, on ajoute 30 ml de soude 10 N puis on chauffe à reflux pendant 4 h sous agitation. Après avoir évaporé le méthanol, le résidue est repris dans un mélange eau-éther, on extrait 3 fois à l'éther, lave 3 fois avec une solution saturée de chlorure de sodium.

Les phases éthérees sont ensuite séchées sur sulfate de magnésium et évaporées à sec. Les cristaux formés sont triturés dans l'hexane, filtrés, lavés à l'hexane et séchés sous vide au dessiccateur.

On obtient 48,8 g de cristaux, légèrement jaunes; Fc = 53—55°C.

d) — CM 40841.

En opérant comme dans l'exemple 1, on prépare ensuite les composés suivants:

— (Hydroxy-4 butyloxy)-4 aniline.
Fc = 56—58°C.

— (Hydroxy-4 butyloxy)-4 benzonitrile.
Fc = 54—58°C.

— CM 40841.
Fc = 143—145°C après recristallisation dans l'éther.

Exemple 6

*Acide (hydroxy-4 butyl)-2 benzoïque (SR 42452)*

A une solution de 22,6 g de diisopropylamine dans 200 ml de tétrahydrofuranne et 28 ml d'hexaméthylphophoramide, on ajoute goutte à goutte à 0°C 140 ml d'une solution 1,6 molaire de butyllithium dans l'hexane. On ajoute ensuite à 0°C la suspension de 15,1 g d'acide méthyl-2 benzoïque dans 50 ml de tétrahydrofuranne puis on agite le mélange pendant 1 h en maintenant la température à 0°C.

On abaisse la température à −5°C et ajoute goutte à goutte 29,7 g de (iodo-3 propoxy)-2 tétrahydro 2H pyrane. On agite pendant 1 h à 0°C puis on ajoute de l'eau et extrait avec de l'éther. On sépare la phase aqueuse qu'on acidifie et extrait avec de l'éther. On sèche la phase organique sur sulfate de sodium et évapore le solvant sous vide.

Les résidu huileux est dissous dans le méthanol; on ajoute 120 ml de solution aqueuse 2 N d'acide chlorhydrique et agite pendant 10 min. On évapore les solvants sous vide, reprend le résidu dans l'eau et extrait avec de l'éther. On lave la solution éthérée 2 fois avec de l'eau, sèche sur sulfate de sodium et évapore les solvants sous vide.

On chromatographie le résidu sur silice en éluant avec du dichloroéthane.

On obtient 5 g du produit attendu; F : 57—9°C.

En utilisant des procédés de préparation analogues, on prépare les composés, selon l'invention, décrits dans le tableau I ci-dessous. Ils sont caractérisés par leur spectre de RMN ou leur point de fusion mesuré après recristallisation dans un mélange éthanol hexane ou dans l'hexane seul pour le SR 41614.

TABLEAU I

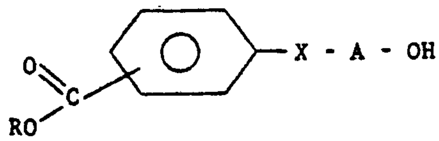

(I)

| n° Produit | Position | X | A - OH | R | Préparé selon exemple | Point de fusion |
|---|---|---|---|---|---|---|
| SR 41576 | p | - | $(CH_2)_4OH$ | H | 1 | 75-80°C |
| CM 40714 | p | O | $(CH_2)_3OH$ | H | 5 | 129-131°C |
| SR 41577 | m | O | $(CH_2)_4OH$ | H | 5 | 105-107°C |
| SR 41614 | o | O | $(CH_2)_4OH$ | H | 5 | 51-53°C |
| SR 41462 | p | - | $(CH_2)_4OH$ | $C_2H_5$ | 2 | RMN |
| CM 40715 | p | O | $(CH_2)_3OH$ | $C_2H_5$ | 5 | RMN |
| CM 40842 | p | O | $(CH_2)_4OH$ | $C_2H_5$ | 5 | RMN |
| SR 42410 | p | - | $(CH_2)_4OH$ | $C_2H_4OH$ | 1 | 57-60°C |
| SR 42791 | o | - | $(CH_2)_4OH$ | $C_2H_5$ | 2 | RMN |
| SR 42900 | m | O | $(CH_2)_4OH$ | $C_2H_5$ | 2 | RMN |
| SR 42913 | p | - | $-C(CH_3)_2CH_2OH$ | $C_2H_5$ | 2 | RMN |

Spectres de RMN des produits selon l'invention:

— SR 41462:
3H à 1,3 ppm (T, J = 7 Hz, $—CO_2—CH_2—C\mathit{H}_2$)
4H entre 1,4 et 1,8 ppm (massif, $HO—CH_2—C\mathit{H}_2—C\mathit{H}_2—CH_3—$)
1H à 1,9 ppm (S, $\mathit{H}O—$)
2H à 2,6 ppm (T, J = 7 Hz, $—CH_2—CH_2—CH_2—C\mathit{H}_2—C_6H_4$)
2H à 3,6 ppm (T, J = 6 Hz, $HO—C\mathit{H}_2—CH_2—CH_2—CH_2—$)
2H à 4,3 ppm (Q, J = 7 Hz, $—CO_2—CH_2—CH_3$)
2H à 7,3 ppm (D, J = 9 Hz, H ortho $CH_2$)
2H à 8 ppm (D, J =9 Hz, H ortho $CO_2$).

— CM 40715:
3H à 1,3 ppm (T, J = 7 Hz, $—CO_2—CH_2—CH_3$)
4H entre 1,5 et 2,2 ppm (massif, $HO—CH_2—C\mathit{H}_2—C\mathit{H}_2—CH_2—O—$)
3H entre 3,5 et 3,9 ppm (massif, $—CH_2—C\mathit{H}_2—O—C_6H_4$, $\mathit{H}O$)
2H à 4,1 ppm (T, J = 6 Hz, $HO—C\mathit{H}_2—CH_2—$)
2H à 4,3 ppm (Q, J = 7 Hz, $—CO_2—C\mathit{H}_2—CH_3$)
2H à 6,9 ppm (D, J =9 Hz, H méta $CO_2$)
2H à 8 ppm (D, J =9 Hz, H ortho $—CO_2$)

— CM 40842:
3H à 1,3 ppm (T, J = 7 Hz, $—CO_2—CH_2—C\mathit{H}_3$)
2H entre 1,7 et 2,3 ppm (M, $—CH_2—C\mathit{H}_2—CH_2—O—C_6H_4$)
2H à 3,8 ppm (T, J = 6 Hz, $—CH_2—C\mathit{H}_2—O—C_6H_4$)
2H à 4,1 ppm (T, J = 6 Hz, $HO—C\mathit{H}_2—CH_2—CH_2$)
2H à 4,3 ppm (Q, J = 7 Hz, $—CO_2—C\mathit{H}_2—CH_3$)
2H à 6,9 ppm (8D, J = 9 Hz, H méta $CO_2$)
2H à 8 ppm (D, J = 9 Hz, H ortho $CO_2$).

— CM 42791:
3H à 1,3 ppm (T, J = 7 Hz, $—CO_2CH_2C\mathit{H}_3$)
4H entre 1,35 et 1,85 ppm (M, $HO—CH_2—C\mathit{H}_2—C\mathit{H}_2—CH_2—$)
1H à 1,95 ppm (S, $O\mathit{H}$)
2H à 2,9 ppm (T, J = 6 Hz, $—CH_2—C\mathit{H}_2—C_6H_4$)
2H à 3,62 ppm (T, J = 6 Hz, $HOC\mathit{H}_2—CH_2$)

2H à 4,27 ppm (Q, J = 7 Hz, $—CO_2CH_2CH_3$)
3H entre 7,0 et 7,6 ppm (M, H aromatique, méta et para $CO_2$)
1H à 7,85 ppm (D de D, J1 = 8 Hz, J2 = 2 Hz, H ortho $CO_2$)
— CM 42900:
3H à 1,3 ppm (T, J = 7 Hz, $CO_2CH_2C\mathit{H}_3$)
5H entre 1,5 et 2,1 ppm (M, $\mathit{H}OCH_2—C\mathit{H}_2—C\mathit{H}_2—CH_2—$)
2H à 3,68 ppm (T, J = 6 Hz, $HO—C\mathit{H}_2CH_2$)
2H à 3,98 ppm (T, J = 6 Hz, $O—C\mathit{H}_2CH_2—$)
2H à 4,27 ppm (Q, J = 7 Hz, $CO_2C\mathit{H}_2CH_3$)
4H entre 6,83 et 7,8 ppm (M, H aromatiques)
— CM 42913:
6H à 1,28 ppm (S, 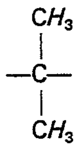
3H à 1,3 ppm (T, J = 7 Hz, $CO_2CH_2C\mathit{H}_3$)
1H à 1,53 ppm (S élargi, O*H*
2H à 3,57 ppm (S, $C\mathit{H}_2OH$)
2H à 4,29 ppm (Q, J = 7 Hz, $CO_2C\mathit{H}_2CH_3$)

| | | |
|---|---|---|
| 2H à 7,4 ppm | système AA', BB' | 2H aromatiques méta $CO_2$ |
| 2H à 7,9 ppm | | 2H aromatiques ortho $CO_2$ |

L'activité bactéricide des produits selon l'invention a été étudiée sur différentes souches par la méthode décrite ci-dessous:

Un inoculum bactérien est mis en contact avec différentes dilutions du produit à tester, et ce pendant un temps limité. A la fin du contact, une partie aliquote du mélange suspension bactérienne/produit est déposée à la surface d'un milieu de culture gélosé contenant un neutralisant de l'activité antibactérienne du produit.

La concentration bactéricide retenue est la concentration minimale de produit à partir de laquelle les bactéries ne poussent plus. Cette concentration est exprimée en µg/ml.

Les souches bactériennes choisies pour l'étude sont:

1 — Escherichia Coli CNCM 54125;
2 — Klebsiella pneumoniae capsulée R030;
3 — Pseudomonas aeruginosa CMCM A22;
4 — Streptococcus faecalis CNCM 5855;
5 — Staphylococcus aureus CNCM 53154.

La deuxième est entretenue sur milieu Worgel Fergusson, les autres sur Tryptic Soy Agar-Difco (TSA).

Après 24 h de culture à 37°C, on récolte la pousse microbienne à l'aide de billes de verre et de 10 ml de diluant contenant 1 g de tryptone et 8,5 g de chlorure de sodium dans 1 000 ml d'eau distillée. On agite la suspension formée et on mesure au spectrophotomètre le pourcentage de transmission de la lumière à 620 nm:

— Souche 1 : 70%;
— Souche 2 : 80%;
— Souche 3 : 70%;
— Souche 4 : 60%;
— Souche 5 : 60%.

L'inoculum bactérien correspond à une suspension au 1/20e de cette suspension bactérienne.

Des plaques comportant des cupules reçoivent différentes dilutions du produit à étudier. Ces dilutions du produit à étudier sont mises en contact avec les différentes suspensions bactériennes à l'aide d'un inoculateur à site multiple type Steers. Après 20 min de contact, des parties aliquotes sont transférées à l'aide de cet inoculateur à la surface d'un milieu gélosé (TSA) placé dans des boîtes de Petri, contenant un neutralisant de l'activité, à savoir 20 g de Lubrol® W, 25 g de Tween 80® et 2,5 g de thiosulfate de sodium dans 1 000 ml de TSA (Difco). Un témoin de l'efficacité du neutralisant est réalisé pour chaque produit étudié en déposant à la surface du milieu de culture une partie aliquote de la dilution du produit à étudier. Après séchage, l'inoculum correspondant est déposé au même endroit. Un témoin inoculum est réalisé sur milieu gélosé avec et sans neutralisant. La lecture se fait après 48 h d'incubation à 37°C.

Les résultats sont rassemblés dans le tableau II ci-dessous.

TABLEAU II

Concentration minimale bactéricide (CMB) en µg/ml

| N° du produit | Souches bactériennes | | | | |
|---|---|---|---|---|---|
| | 1 | 2 | 3 | 4 | 5 |
| SR 41323 | 1 500 | 500 | 500 | 3 000 | 1 500 |
| SR 41576 | 500 | 500 | 100 | 1 000 | 500 |
| CM 41074 | 2 000 | 500 | 1 000 | 2 000 | 2 000 |
| CM 40714 | 1 000 | 1 000 | 500 | 1 000 | 2 000 |
| CM 40841 | 2 000 | 1 000 | 500 | 2 000 | 1 000 |
| SR 41577 | 500 | 200 | 200 | 1 000 | 500 |
| SR 41614 | 500 | 500 | 200 | 1 000 | 500 |
| CM 40715 | 1 000 | 2 000 | 500 | 2 000 | 2 000 |
| CM 40842 | 1 000 | 1 000 | 500 | 2 000 | 2 000 |
| SR 41462 | 500 | 500 | 500 | 800 | 500 |
| CM 41075 | 500 | 1 000 | 1 000 | 2 000 | 2 000 |
| SR 42452 | 1 000 | 1 000 | 500 | 1 000 | 1 000 |
| SR 42900 | 1 000 | 500 | 500 | 1 000 | 500 |
| SR 41324 | 600 | 600 | 600 | 2 500 | 2 000 |
| SR 42791 | 1 500 | 1 000 | 500 | 1 500 | 1 500 |
| SR 42913 | 500 | 500 | 100 | 1 000 | 500 |

Les résultats montrent que les produits selon l'invention présentent un large spectre d'activité sur les souches bactériennes testées.

L'activité antifongique des produits selon l'invention a aussi été déterminée selon la méthode des dilutions sériées en milieu gélosé.

Des solutions mères des différents produits sont réalisées à l'aide de tétraglycol à 20%. A partir de ces solutions mères, on réalise un gamme de dilutions selon une progression géométrique de raison 1/2. 2 ml de chacune de ces dilutions sont distribués dans des boîtes de Petri et on y ajoute 1,8 ml de milieu gélosé "Sabouraud Dextrose Agar" (Difco).

L'inoculum est constitué pour les levures d'une dilution au 1/20 d'une culture de 48 h à 27°C en milieu liquide de Sabouraud. Pour les dermatophytes et les contaminants, l'inoculum est préparé par la récolte, à l'aide de 5 ml de milieu de Sabouraud liquide, d'une culture en milieu gélosé de Sabouraud.

L'inoculum est déposé à la surface des boîtes de Pétri à l'aide d'un inoculateur à sites multiples. La lecture se fait après 48 h d'incubation à 27°C pour les levures, après 7 jours d'incubation à 27°C pour les dermatophytes et après 4 jours d'incubation à 27°C pour les contaminants. La concentration minimale inhibitrice ou CMI exprimée en µg/ml représente la plus petite concentration pour laquelle aucune pousse ne s'est produite.

Le tableau III rassemble les résultats obtenus avec différents produits selon l'invention.

Les expériences ont été effectuées avec 22 souches de levures, 18 souches de dermatophytes et 8 souches de contaminants. Les résultats (CMI en µg/ml) représentent les valeurs extrêmes obtenues pour chaque catégorie.

TABLEAU III

Activité antifongique

| N° du produit | Levures | Dermatophytes | Contaminants |
|---|---|---|---|
| SR 41462 | 300-750 | 9-38 | 300-750 |
| SR 41577 | 1 500 | 150 | 1 500 |
| CM 41075 | 500-1 000 | 62,5 | 250-1 000 |
| CM 40715 | 500-1 000 | 62,5-125 | 500-1 000 |

Ces résultats montrent que les produits selon l'invention possèdent une bonne activité vis-à-vis des champignons étudiés.

La tolérance des produits selon l'invention a été étudiée chez le cobaye. Les animaux sont tondus de part et d'autre de la ligne médiane du dos, la tonte est entretenue tous les 2 jours. Des lots de 6 animaux reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée préliminaire, le traitement est appliqué 1 fois par jour, 6 jours sur 7, durant 3 semaines. Les observations sur le plan cutané portent sur la présence d'érythème, d'éruption cutanée ou d'hyperkératose dont l'intensité est graduée selon un barème déterminé.

L'essai de sensibilisation cutanée est réalisé sur les mêmes animaux après deux semaines de repos. Le traitement dure une semaine, il est identique au précédent. L'évaluation se fait sur les mêmes critères et d'aprés le même barème que celui utilisé pour la tolérance locale.

On a également recherché si les produits selon l'invention présentent un effet phototoxique ou photoallergique chez le cobaye. La technique mise en oeuvre est celle de UNKOVIC J. MAZUE G. et GIRARD J., dans Sciences et Techniques de l'Animal de laboratoire vol. 8 (3), 149—160, (1983). C'est une adaptation des techniques décrites par HARBER L.C. et al, Arch. Dermatol., 1967, vol. 96, p. 646—656 et VINSON L.J. et al, J. Soc. Cosm. Chem., 1966, vol. 17, p. 123—130.

Aucun des produits étudiés n'a montré mauvaise tolérance, d'effet sensibilisant ni d'effet phototoxique ou photo-allergique chez le cobaye.

Une évaluation de la toxicité aiguë par voie orale a été réalisée chez la souris. Cette étude a été effectuée sur des souris mâles de souche CD1 prevenant de l'élevage Charles River. Chaque lot était composé de 5 animaux d'un poids corporel variant de 24 à 30 g entretenus dans une même cage.

Les animaux étaient conservés à jeun pendant 6 h avant le traitement. Pour chaque étude, le produit mis en suspension dans une solution de gomme arabique à 10% a été administré par gavage à l'aide d'une sonde oesophagienne.

La nourriture était de nouveau distribuée aux animaux 4 h après le gavage et les animaux étaient gardés en observation pendant une période de 14 jours après l'administration.

Pendant cette période, on note la mortalité dans chacun des lots mis en expérience et lorsque cela est possible on détermine la dose létale 50 (DL 50) en utilisant la méthode de Litchfield et Wilcoxon.

Les résultats suivants, exprimés en mg de substance essayée par kg de poids corporel, ont été obtenus.

SR 41 323 DL 50>50 000

SR 41 576 DL 50 entre 4 000 et 5 000

Les composés selon l'invention présentent un niveau d'activité intéressant qui se compare favorablement à celui des principales familles d'antiseptiques utilisés dans la pratique.

En outre les produits selon l'invention présentent une activité homogène vis-à-vis des différentes espèces bactériennes étudiées. De plus, ils sont dotés d'une faible toxicité, ils ont montré une bonne tolérance et sont dépourvus d'effet sensibilisant et d'effet phototoxique ou d'effet photoallergique.

Par suite, les produits selon l'invention pourront recevoir de multiples applications comme antiseptiques, conservateurs ou désinfectants.

En particulier, ils pourront être utilisés comme antiseptiques dans les préparations à visées thérapeutiques, par exemple, dans le traitement de l'impétigo, l'acné, les dermatoses infectées, les plaies ouvertes infectées, les infections fermées telles que furoncles, panaris, gales impétiginisées etc. On peut également envisager une utilisation à visée préventive, par exemple, pour la préparation du champ chirurgical, la préparation des mains du chirurgien ou du personnel soignant.

En application vétérinaire, les produits selon l'invention peuvent être utilisés soit comme antiseptiques (par exemple dans la prévention des mammites) soit comme désinfectants (désinfection des étables, du matériel . . .) de même que dans le domaine agroalimentaire.

Enfin, leur bonne tolérance et leur faible toxicité les autorisent à être utilisés comme conservateurs, non seulement dans le domaine pharmaceutique et cosmétologique mais également dans le domaine agroalimentaire.

Différentes formulations galéniques des produits selon l'invention peuvent être préparées en fonction de l'application choisie.

Exemple 7

| | | | |
|---|---|---|---|
| Soluté antiseptique alcoolique SR 41462 | | 0,5 | g |
| Alkyldiméthylcarboxyméthylamine (solution à 30%) | | 0,5 | g |
| Condensat d'oxyde d'éthylène et de propylèneglycol L62 | | 1 | g |
| Acide lactique ou Hydroxyde de de sodium | qsp pH 6,5 | | |
| Alcool éthylique à 70° | qsp | 100 | g |

Exemple 8

Un produit selon l'invention peut être utilisé comme conservateur dans un shampooing:

| | | | |
|---|---|---|---|
| Palmitate de potassium et d'acides aminés | | 20 | g |
| Alkylsulfates de sodium | | 2 | g |
| Diéthanolamide de coprah | | 5 | g |
| Acétate de linalyle | | 0,200 | g |
| CM 41075 | | 0,150 | g |
| Hydroxyde de sodium | qsp pH 7 | | |
| Eau purifiée | qsp | 100 | g |

Exemple 9

Un produit selon l'invention peut être utilisé comme conservateur dans une crème émulsion.

| | | | |
|---|---|---|---|
| Huile de Vaseline® épaisse | | 6 | g |
| Mélange d'alcool cétostéarylique et d'alcool cétostéarylique oxyméthyléné | | 9 | g |
| Phosphate monosodique anhydre | | 0,300 | g |
| Tétracémite disodique | | 0,010 | g |
| Vaseline® | | 15 | g |
| CM 41075 | | 0,100 | g |
| Acide phosphorique | qsp pH 4,5 | | |
| Eau purifiée | qsp | 100 | g |

Exemple 10

Un produit selon l'invention peut être utilisé comme conservateur dans une crème pour utilisation cosmétologique.

| | | | |
|---|---|---|---|
| Collagène | | 0,500 | g |
| Carboxypolyméthylène 934 | | 0,400 | g |
| Lanoline hydrogénée | | 4 | g |
| Perhydrosqualène | | 20 | g |
| Monopalmitate de sorbitol polyoxyméthyléné | | 2 | g |
| SR 42913 | | 0,150 | g |
| Acide lactique ou hydroxyde de sodium | qsp pH 6,5 | | |
| Eau purifiée | qsp | 100 | g |

Exemple 11

Préparation antiseptique liquide détergente moussante:

| | | | |
|---|---|---|---|
| CM 41075 | | 0,3 | g |
| Alkyldiméthylcarboxyméthylamine (solution à 30%) | | 15 | g |
| Tétracémate disodique | | 0,1 | g |
| Propylèneglycol | | 20 | g |
| Hydroxyde de sodium | qsp pH 5,8 | | |
| Eau purifiée | qsp | 100 | g |

Exemple 12

Préparation antiseptique liquide détergente moussante:

| | | | |
|---|---|---|---|
| SR 42913 | | 0,2 | g |
| Paraffine sulfonate de sodium | | 15 | g |
| Hydroxyde de sodium ou acide lactique | qsp pH 5,2 | | |
| Eau purifiée | qsp | 100 | g |

Exemple 13

Désinfectant pour surface inerte:

| | | | |
|---|---|---|---|
| SR 41576 | | 0,5 | g |
| Dodécyldiméthylcarboxydiméthylamine | | 20 | g |
| Tétracémate disodique | | 2 | g |
| Acide lactique | qsp pH 3,5 | | |
| Eau purifiée | qsp | 100 | g |

Exemple 14

Conservateur pour jus de fruits ou confiture:

| | | |
|---|---|---|
| SR 41576 | micronisé | 0,05 % |

Exemple 15

Conservateur pour crèmes:

| | | |
|---|---|---|
| SR 42900 | micronisé | 0,05 % |

**Revendications**

1. Dérivés de l'acide benzoïque, de formule

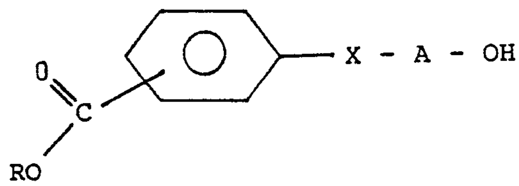

(I)

dans laquelle:
— A représente une chaîne alkylene droite ou ramifiée comprenant de 3 à 4 atomes de carbone;
— X représente l'atome d'oxygène ou une liaison directe;
— R représente l'hydrogene ou un groupement alkyle de 2 à 6 atomes de carbone éventuellement substitué par une fonction alcool; ou un de leurs sels, pour utilisation comme médicament, à usage humain ou vétérinaire.

2. Dérivés de l'acide benzoïque selon la revendication 1, pour utilisation comme agent antimicrobien.

3. Dérivés de l'acide benzoïque selon l'une des revendications 1 ou 2, de formule (I) dans laquelle X est une liaison directe, R est l'hydrogène, le groupe $C_2H_5$ ou $C_2H_4OH$ et A—OH est choisi parmi les groupes $(CH_2)_3OH$; $(CH_2)_4OH$; $(CH_2)_2CH(OH)CH_3$.

4. Dérivés de l'acide benzoïque selon l'une des revendications 1 ou 2, de formule (I) dans laquelle X est l'oxygène, R est l'hydrogène ou le groupe éthyle et A—OH est $(CH_2)_3OH$ ou $(CH_2)_4OH$.

5. Utilisation des dérivés de l'acide benzoïque de formule (I) selon la revendication 1, à titre d'agents conservateurs dans le domaine pharmaceutque, cosmétique ou agroalimentaire.

6. Utilisation des dérivés de l'acide benzoïque de formule (I) selon la revendication 1, à titre de désinfectants de surface inerte.

**Patentansprüche**

1. Benzoesäurederivate der Formel

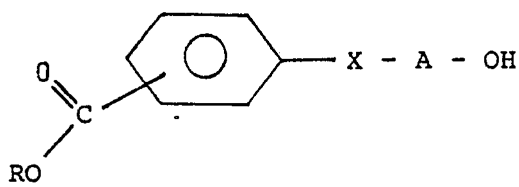

(I)

worin:
— A eine gerade oder verzweigte Alkylengruppe mit 3 bis 4 Kohlenstoffatomen darstellt;
— X ein Sauerstoffatom oder eine direkte Bindung bedeutet;
— R für Wasserstoff oder eine gegebenenfalls durch eine Alkoholfunktion substituierte Alkylgruppe mit 2 bis 6 Kohlenstoffatomen steht;
oder eines ihrer Salze zur Verwendung als Medikament für humanen oder veterinären Gebrauch.

2. Benzoesäurederivate nach Anspruch 1 zur Verwendung als Bakteriostatikum.

3. Benzoesäurederivate nach einem der Ansprüche 1 oder 2 der Formel (I), worin X eine direkte Bindung ist, R für Wasserstoff, die Gruppe $C_2H_5$ oder $C_2H_4OH$ steht und A—OH ausgewählt ist aus den Gruppen $(CH_2)_3OH$; $(CH_2)_4OH$; $(CH_2)_2CH(OH)CH_3$.

4. Benzoesäurederivate nach einem der Ansprüche 1 oder 2 der Formel (I), worin X Sauerstoff ist, R Wasserstoff oder eine Ethylgruppe bedeutet und A—OH für $(CH_2)_3OH$ oder $(CH_2)_4OH$ steht.

5. Verwendung der Benzoesäurederivate der Formel (I) nach Anspruch 1 als Konservierungsmittel auf dem Gebiet der Pharmazie, Kosmetik oder Agrar-Nahrungsmittelindustrie.

6. Verwendung der Benzoesäurederivate der Formel (I) nach Anspruch 1 als Desinfektionsmittel für inerte Oberflächen.

## Claims

1. Benzoic acid derivatives, of formula

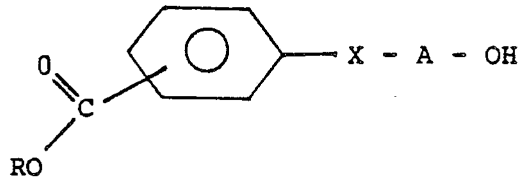

(I)

in which:
A represents a straight or branched alkyl chain with 3 to 4 carbon atoms,
X represents the oxygen atom or a direct bond,
R represents hydrogen or an alkyl group with 2 to 6 carbon atoms, possibly substituted by an alcohol function;
or one of their salts for use as medicine, for veterinary or human use.

2. Benzoic acid derivatives according to claim 1, for use as antimicrobial agent.

3. Benzoic acid derivatives according to any one of claims 1 or 2, of formula (I) in which X is a direct bond, R is hydrogen, the $C_2H_5$ or $C_2H_4OH$ group and A—OH is selected among the groups $(CH_2)_3OH$; $(CH_2)_4OH$; $(CH_2)_2CH(OH)CH_3$.

4. Benzoic acid derivatives according to any one of claims 1 or 2, of formula (I) in which X is oxygen, R is hydrogen or the ethyl group and A—OH is $(CH_2)_3OH$ or $(CH_2)_4OH$.

5. Use of the benzoic acid derivatives of formula (I) according to claim 1, as preserving agents in the pharmaceutical, cosmetic or agrifoodstuffs field.

6. Use of the benzoic acid derivatives of formula (I) according to claim 1, as inert surface disinfectants.